# EUROPEAN PATENT SPECIFICATION

(11) **EP 2 552 364 B1**
(45) Date of publication and mention of the grant of the patent: **13.05.2015**
(21) Application number: 11716619.9
(22) Date of filing: 22.03.2011
(51) Int. Cl.: A61F 5/058, A61F 5/01

(54) **FRACTURE BRACE**
BRUCHBANDAGE
APPAREIL ORTHOPÉDIQUE POUR FRACTURE

(30) Priority: 01.04.2010 GB 201005566
(43) Date of publication of application: 06.02.2013
(73) Proprietor: Aberdeen Orthopaedics Developments Limited, Cults Aberdeen, AB15 9NX (GB)
(72) Inventor: WARDLAW, Douglas, Stonehaven Aberdeenshire AB39 3QR (GB)
(74) Representative: Allan, James Stewart
(86) International application number: PCT/GB2011/050565
(87) International publication number: WO 2011/121332

(56) References cited:
- EP-A1- 0 255 388
- WO-A1-01/43671
- WO-A2-2007/050703
- US-A- 5 527 265
- US-A1- 2004 019 306
- US-A1- 2006 052 730

## Description

This invention relates to the treatment of fractures, and it relates especially, although not exclusively, to apparatus for effecting such treatments as are applicable to fractures of long bones such as the radius. Fractures of the lower end of the radius called Colles' fractures are particularly suitable. Such fractures result in swelling of the injured part which tends to gradually subside as the fracture heals.

The standard treatment for Colles Fractures throughout the world is a so-called Colles Plaster of Paris cast. Usually the cast is applied at the time of fracture reduction and then changed after ten days to two weeks to a definitive cast for a further four weeks or so. This immobilises the wrist and leaves a very stiff wrist which takes several months to recover. Also, this method of treatment is sometimes not successful in maintaining the fracture position and can leave significant long-term deformity of the wrist. This fracture occurs most commonly in elderly people and during the period of cast treatment and subsequent rehabilitation they have very little proper use of the hand. Many of them live on their own and as a result their ability to look after themselves is significantly impaired in the short term.

An alternative to a Plaster of Paris cast is to use a fracture brace, for example as disclosed in EP 0 255 388. This fracture brace is a rigid brace comprising two main portions, conjoined together by two, three or four elasticated Velcro straps. The first portion covers the dorso-radial surfaces of the forearm from the level of the radio-carpal joint and radial styloid for approximately two thirds of the forearm. At either end of the brace, there are two areas of high loading of a specified size, which are raised by approximately 5 millimetres. The second portion of the brace is applied to the antero-ulnar aspect of the forearm and has a similar raised area of a specified size. When the brace is applied, the straps are tightened around the two portions, imparting a force on the fracture by means of the three point loading. The elastic section of the strap is stretched with the intention that the energy stored in the stretched elastic is released so that a degree of loading on the fracture is maintained.

Clinical trials carried out in Aberdeen showed that the concept of this fracture brace was correct. Using this fracture brace instead of a conventional cast allowed earlier functioning of the limb, and resulted in the pinch-grip (the ability to hold objects between the finger and thumb) and the grip strength returning earlier than would otherwise happen.

Unfortunately when multi-centre clinical trials were carried out, optimum results were not achieved. One problem was that the elastic straps did not maintain adequate loading on the fracture. When first applied to a fracture, the limb tends to be quite swollen and the elastic straps stretch to accommodate the swollen limb. However, as the swelling gradually subsides, the straps tend to "sag", rather than return fully to their original unstretched shape. Hence, the brace was unable to maintain adequate fracture loading.

Another problem was the fracture brace of EP 0 255 388 failed to maintain the fracture position adequately enough to allow a significant long term advantage. Thus, the long term results were similar to those obtained from using a conventional cast.

An additional problem was that there was a small risk of compressing the limb circumferentially as does a tourniquet. Intra-cast pressure measurements carried out within this brace compared to a Plaster of Paris cast demonstrated that pressures within the brace were significantly greater than in a standard cast used for treating these fractures especially the pressures under the loaded areas. A potential complication of a cast or brace treatment is that after application, the swelling due to the facture could increase, especially in a situation where the limb was not held initially in a support and was allowed to simply hang by the side. In this situation, swelling could increase to the point where the circulation the limb is impeded with serious consequences. Some of the pressures under the fracture brace of EP 0 255 388, although less than under the loaded areas, were still significantly higher than in the cast and theoretically may in some cases have resulted in compression of the limb similar to a tourniquet. A reduction in the loading applied was designed into the brace to compensate for this, and may have resulted in a failure of the device to control the fracture position in some cases.

Also there was a concern that pressures greater than sixty five degrees of mercury might cause skin necrosis causing pressure sores as occurs in debilitated patients. However, it is known that pressure sores occur as a result of both pressure and a shear force on the skin, mostly the latter, often combined with loss of sensation to the part and poor general nutrition. None of these additional factors are present in patients treated by the brace which applies pressure to the fracture through the skin and overlying soft tissues. The pressure is applied by a soft pad over a relatively wide area and is dissipated through the soft tissues so that a high pressure point that would cause a local pressure area cannot occur. A high pressure point sufficient to cause skin necrosis would be extremely painful requiring the brace to be removed before such necrosis occurred.

An alternative fracture brace is disclosed in WO 01/43671. This brace also comprises two portions joined together by an elasticated strap. The first portion has one preformed pad and the second portion has two preformed pads joined together by two curved, resilient struts. The elasticated strap stretches around the single pad of the first portion, contacts the limb on both sides, and then extends over the two resilient struts of the second portion. The resilient struts and the elasticated strap both deform when the brace is applied; specifically the strap stretches and the resilient struts tend to straighten out. As the swelling goes down, the intention was that the resilient struts should revert to their more curved position, to maintain pressure on the fracture.

However, a problem that was discovered in clinical trials is that the elastic strap did not maintain adequate loading on the fracture. Specifically, when the brace is applied, the stretching of the elastic strap meant that the resilient struts were not caused to straighten out to a sufficient extent. Hence, in practice, only a small amount of energy was stored in the resilient struts. Given that the resilient struts did not deform much in the first place, they could not deform back again very far upon the decrease of swelling of the fracture. Furthermore, when the resilient struts began to straighten out, instead of maintaining pressure on the fracture, the elasticated straps again tended to take the strain, and just tended to stretch further.

Another problem with the fracture brace of WO 01/43671 is that the combination of the elasticated strap and the preformed pad of the first portion together contact about half of the circumference of the limb. This risks an undesirable constriction of the limb.

US2006/0052730 discloses a fracture brace that is also useful for understanding the invention.

According to a first aspect of the present invention there is provided a fracture brace according to claim 1.

By "resilient" we mean that the material returns (at least partially) towards its original form or position after being bent, compressed or stretched.

In use, as the fracture swelling subsides, the resilient material reacts by partially returning towards its original shape, so that any slackening of the strap caused by the reduced swelling is taken up by the resilient material.

The rigidity of the shell ensures that, when the strap is tightened, loading is applied to the limb only from the two load areas of the first portion and from the load area of the second portion. No direct loading is applied to the limb from any of the rest of the shell. Hence, undesirable constriction of the limb is avoided.

Embodiments of the fracture brace of the present invention can provide sustained fracture reduction throughout treatment by means of three point loading through three separate load areas.

Compared to Plaster of Paris casts, embodiments of the present invention can result in improved stability of fractured bones and/ or mobility of joints. Hence, there can be a decreased incidence of deformity and/ or morbidity as bones and joints are allowed free movement in a stabilised manner.

Compared to known fracture braces, embodiments of the present invention can provide enhanced fracture loading throughout the duration of the period of treatment.

Typically, the strap is inelastic.

This ensures that the fracture brace provides even better loading of the fracture. Specifically, the strap being inelastic ensures that the resilient material is sufficiently compressed when the brace is applied, rather than the strap simply stretching. The only component which can take the force of the compression is the resilient material; hence, a large amount of energy can be stored in the resilient material. Furthermore, upon decrease of the swelling, the strap cannot contract so instead, the resilient material must deform back to or towards its original shape. The lack of stretching of the strap ensures that at substantially all times, adequate loading of the fracture is maintained.

The position of the resilient material is typically closer to the distal load area than to the proximal load area to apply more constant loading on the distal part of the limb. In practice, the loading applied to the proximal part of the limb tends to be dissipated more by the soft tissue of the proximal part of the limb, so arranging the resilient material and optionally the strap asymmetrically in this way, closer to the more rigid distal forearm and the distal load area than to the proximal load area, makes the system more effective in maintaining a constant loading. The asymmetric position closer to the distal load area is approximately in the ratio two fifths to three fifths.

The resilient material is typically wider than the strap. Optionally the strap overlies the resilient material on each side of the strap. Typically about 5mm of resilient material extends beyond each side of the strap to resist slippage of the strap away from the resilient material and thereby ensuring that the correct volume of resilient material is compressed by the strap.

Optionally, the strap has an angle creased in it to allow it to conform to the conical shape of a limb.

The creased angle in the strap allows an inelastic strap to lie evenly on the first and second portions of the brace.

Typically, the first and second portions are shaped such as to space the entirety of the strap from the limb in use.

Therefore, no loading is applied directly from the strap to the limb. This prevents undesirable constriction of the limb, avoiding e.g. a tourniquet effect of the strap compressing the limb.

Optionally, the rigid shell is u-shaped in cross-section, the strap extending over and around the u-shape directly to the second portion without touching the limb.

The u-shaped rigid shell helps to hold the strap out of contact with the limb so that no loading is applied directly from the strap to the limb. Hence, undesirable constriction of the limb is avoided.

Typically, the rest of the fracture brace is located radially outwards of the load areas so that, apart from the load areas, no other part of the fracture brace contacts the limb in use.

Hence, no pressure or loading is imparted at any other point on the limb. This also prevents undesirable constriction of the limb.

Typically, each load area comprises an attached preformed pad.

Typically, the preformed pads are made of a high friction material adapted to prevent slippage of the brace and thus maintain its position on the limb in use.

Optionally, the pads are hypoallergenic.

Optionally, the resilient material comprises polyurethane.

Typically, the resilient material comprises a foam or sponge.

Optionally, only two load areas are provided on the first portion and only one load area is provided on the second portion.

Hence, such embodiments provide three and only three points of loading on the limb.

In a typical embodiment, the present invention comprises an almost constant loading force at three points.

Typically the load area on the second portion is located between the two load areas provided on the first portion.

Optionally the strap overlies at least a part of the load area on the second portion and extends between the two load areas provided on the first portion, typically closer to the distal load area than the proximal load area to apply more constant loading on the distal load area.

Typically the two load areas provided on the first portion are spaced away from one another towards opposite ends of the first portion. Embodiments of the invention allow a method of treating a fracture in a limb, comprising:
reducing the fracture;
applying a first portion of a fracture brace to the limb, the first portion comprising a rigid shell having at least two load areas which contact the limb and a resilient material attached to the radially outer part of the shell;
applying a second portion of the fracture brace to the limb, the second portion comprising at least one load area which contacts the limb;
encircling the first and second portions of the fracture brace with a strap;
tightening the strap; and
securing the strap in its tightened position such that the strap overlies and compresses the resilient material, thereby causing the fracture brace to exert pressure on the limb;
wherein the resilient material re-expands upon any decrease of swelling in the limb to maintain pressure on the limb.

The various aspects of the present invention can be practiced alone or in combination with one or more of the other aspects, as will be appreciated by those skilled in the relevant arts. The various aspects of the invention can optionally be provided in combination with one or more of the optional features of the other aspects of the invention. Also, optional features described in relation to one embodiment can typically be combined alone or together with other features in different embodiments of the invention.

Various embodiments and aspects of the invention will now be described in detail with reference to the accompanying figures. Still other aspects, features, and advantages of the present invention are readily apparent from the entire description thereof, including the figures, which illustrates a number of exemplary embodiments and aspects and implementations. The invention is also capable of other and different embodiments and aspects, and its several details can be modified in various respects, all without departing from the spirit and scope of the present invention. Accordingly, the drawings and descriptions are to be regarded as illustrative in nature, and not as restrictive. Furthermore, the terminology and phraseology used herein is solely used for descriptive purposes and should not be construed as limiting in scope. Language such as "including," "comprising," "having," "containing," or "involving," and variations thereof, is intended to be broad and encompass the subject matter listed thereafter, equivalents, and additional subject matter not recited, and is not intended to exclude other additives, components, integers or steps. Likewise, the term "comprising" is considered synonymous with the terms "including" or "containing" for applicable legal purposes.

Any discussion of documents, acts, materials, devices, articles and the like is included in the specification solely for the purpose of providing a context for the present invention. It is not suggested or represented that any or all of these matters formed part of the prior art base or were common general knowledge in the field relevant to the present invention.

In this disclosure, whenever a composition, an element or a group of elements is preceded with the transitional phrase "comprising", it is understood that we also contemplate the same composition, element or group of elements with transitional phrases "consisting essentially of', "consisting", "selected from the group of consisting of', "including", or "is" preceding the recitation of the composition, element or group of elements and vice versa.

All numerical values in this disclosure are understood as being modified by "about". All singular forms of elements, or any other components described herein are understood to include plural forms thereof and vice versa.

An embodiment of the invention will now be described, by way of example only, with reference to the accompanying drawings, in which:
Fig 1 shows a perspective view from above of a fracture brace according to the present invention;
Fig 2 shows a perspective view from the side of the fracture brace of Fig 1;
Fig 3 shows a perspective view of the fracture brace of claim 1 fitted to a patient's arm, seen from the front of the arm;
Fig 4 a further perspective view of the fracture brace of claim 1 fitted to a patient's arm, seen from the back of the arm;
Fig 5 shows a cross-section of the brace on the arm though the distal (lower) section of the brace; and
Fig 6 shows a cross-section of the brace on the arm though the middle section of the brace.

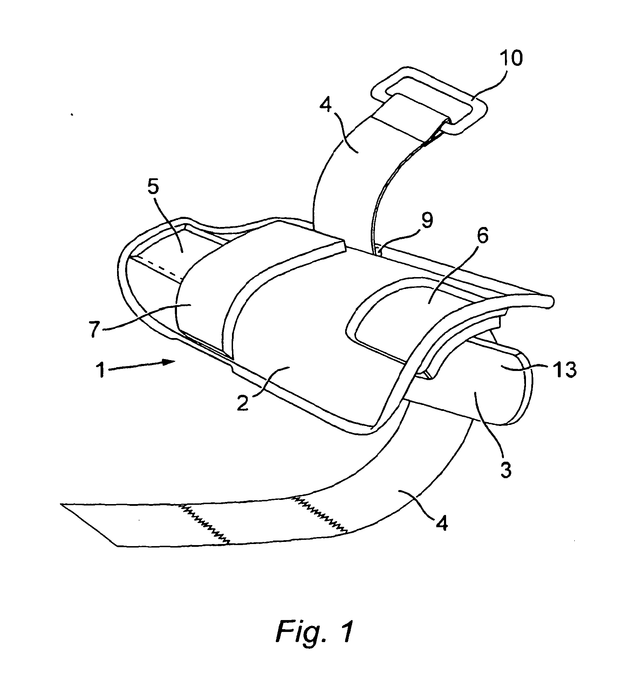

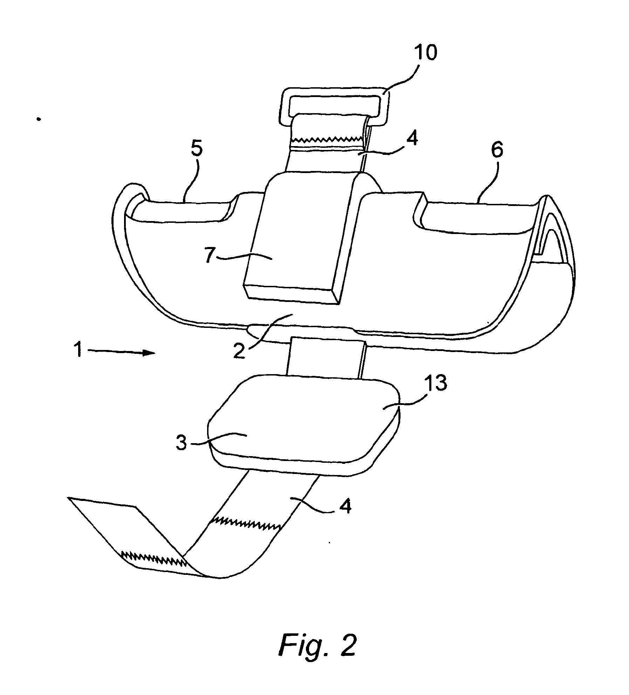

Referring now to Figs 1 to 4, a fracture brace 1 comprises a dorso-radial portion and an antero-ulnar portion 3 joined together by an inelastic Velcro™ strap 4.

The dorso-radial portion comprises a rigid shell 2 that is shaped in an open "U" or "V" with a curved base. The rigid shell 2 is typically made from 3mm thick natural polypropylene (or co-polymer). The rigid shell 2 has two areas of high loading: a distal load area 5 and a proximal load area 6. The load areas 5, 6 are raised by approximately 10 millimetres with respect to the rest of the rigid shell 2.

Attached to the radially outer part of the rigid shell 2 is a body of resilient material 7 comprising a polyurethane sponge. The resilient material 7 has a memory and an appropriate shore hardness such that, when it is deformed energy is stored and, when the deforming force is removed, this energy is released as the body of resilient material 7 returns to its original shape. Typically the foam is high density foam, optionally having a density range of 60-80 kg/m³, typically 65-70 kg/m³, a typical CLD Hardness (40%) of around 5-15 kPa min e.g. 10 kPa min, and a typical tensile strength of around 150 kPa min. The nominal elongation at break is typically 80% min. One suitable foam for use in an embodiment of the invention is HLB65S polyether polyurethane foam available from Kay-Metzeler Corporation in Cheshire, UK, although other resilient materials (such as other foams) can be used. The resilient material is positioned closer to the distal load area and positioned such as to enable it to be compressed by the rigid straps. The asymmetric positioning closer to the more rigid distal forearm makes the system more effective. The typical position closer to the distal load area is approximately in the ratio two fifths to three fifths, but other positions that are asymmetric can function adequately.

The antero-ulnar portion 3 of the brace also has a load area 13 which, in this embodiment, is equal to the whole area of the antero-ulnar portion 3. The antero-ulnar portion 3 of the brace is made from 1 mm thick natural polypropylene (or co-polymer).

Each load area 5, 6, 13 comprises an attached preformed pad made of a high friction, hypoallergenic material. The pads provide comfort to the patient and also help to prevent slippage and maintain the position of the brace 1 on the limb.

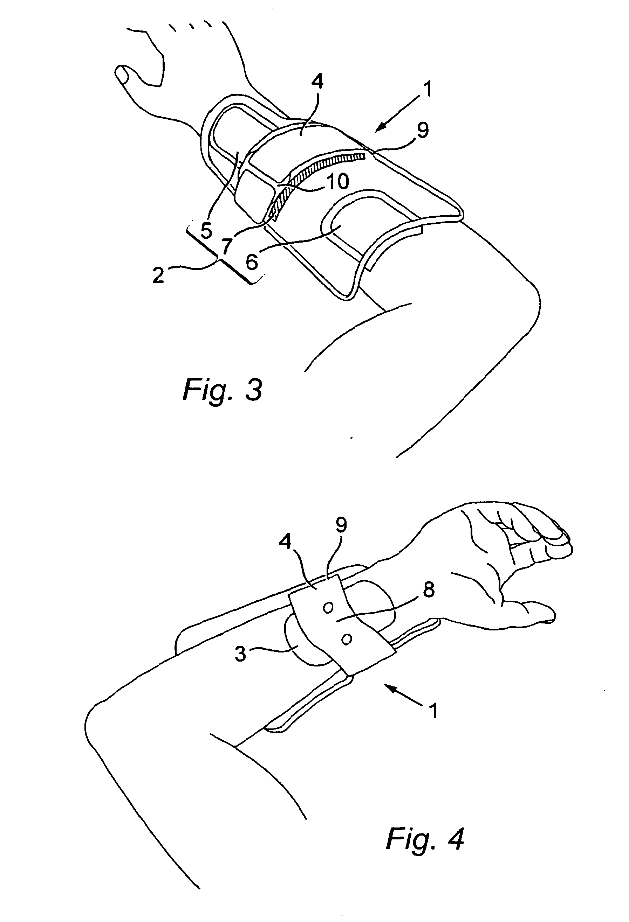

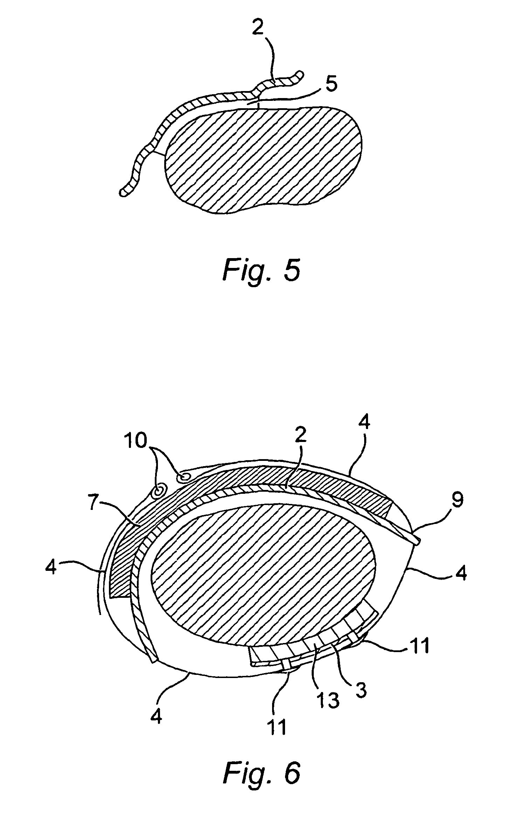

The strap 4 is attached to the antero-ulnar portion 3 by means of rivets 11 (see Fig 6). The strap 4 is provided with a small angulation created at the point 8 (see Fig 4). This helps ensure that the inelastic strap 4 lies evenly when the brace 1 is applied to a (conical) limb.

The strap 4 has a buckle 10 and is threaded through a slot 9 on the dorso-radial portion 2. The strap 4 is typically narrower than the resilient material 7. Typically the strap is around 50 mm wide and the resilient material is around 60 mm wide, so that the strap 4 overlies the resilient material 7which can extend beyond the edges of the strap 4 on each side. The slot 9 is positioned to maintain the strap 4 in a suitable position to overlie the resilient material 7 leaving approximately 5 mm of resilient material on each side of the strap 4. This keeps the strap 4 in place and in register with the resilient material 7 and helps to ensure that the correct volume of resilient material 7 is compressed by the strap 4.

When the brace 1 is fitted to the patient's arm, only the proximal and distal load areas 5, 6 of the dorso-radial portion 2 and the single load area 13 of the antero-ulnar portion 3 are in direct contact with the patient's arm.

This is illustrated particularly in Figs 5 and 6. Fig 5 is a cross-section though the distal (lower) section of the brace 1 and shows the distal load area 5 over the fracture, in direct contact with the patient's arm. Fig 6 shows a cross-section of the brace 1 on the arm though the middle section of the brace 1. In Fig 6, the load area 13 of the antero-ulnar portion 3 contacts the patient's arm, but there is a space between the arm and the rigid shell 2. The rigidity of the shell 2 ensures that the shell 2 cannot be deformed when the strap 4 is tightened and there remains a space between the shell 2 and the skin at all points other than the proximal and distal load areas 5, 6.

Hence, the rest of the fracture brace is located radially outwards of the load areas 5, 6, 13 so that, apart from the load areas 5, 6, 13 no other part of the fracture brace 1 contacts the limb in use. This ensures that no pressure or loading is imparted at any other point on the limb, which prevents undesirable constriction of the limb. Hence, the fracture brace 1 has a shape and structure that ensures that no tourniquet effect can occur, irrespective of how much the brace is tightened.

Fig 6 also illustrates that the two portions of the brace 2, 3 are shaped such as to space the entirety of the strap 4 from the limb in use. Specifically, the strap 4 extends around the U-shape of the rigid shell 2 and passes from the ends of the U-shape directly over to the antero-ulnar portion 3, without any direct contact with the limb. This prevents undesirable constriction of the limb, avoiding e.g. a tourniquet effect of the strap compressing the limb.

In use, the fracture is reduced and the brace is applied immediately after the injury (see Figs 3 and 4). In this example, the fracture (not shown) is a Colles' fracture and would lie beneath the distal load area 5 in Fig 3.

The dorso-radial portion 2 is positioned (see Fig 3) so that it covers the dorso-radial surfaces of the forearm from the level of the radio-carpal joint and radial styloid for approximately two thirds of the forearm. Next, the antero-ulnar portion 3 is applied to the antero-ulnar aspect of the forearm (see Fig 4).

The strap 4 is wrapped around the shell 2 and the antero-ulnar portion 3 so that it encircles the two portions of the brace 1 and overlies the resilient material 7. The strap 4 is passed through the buckle 10 and tightened to a marker on the strap, so that the resilient material 7 is compressed and deformed, which tensions the brace 1. The strap 4 is then secured in this tightened position by the buckle 10.

When the strap 4 is tightened, loading is applied only to the areas of the arm under the pads of the three load areas 5, 6, 13. The rigidity of the shell 2 holds the rest of the shell 2 (apart from the proximal and distal load areas 5, 6) and the strap 4 away from the arm, ensuring that no pressure is exerted on any other parts of the arm. This ensures that there is no tourniquet effect and that zero pressure is present between the skin and brace at any parts other than the three load areas 5, 6, 13. Studies on volunteer subjects have confirmed that this is the case. This is advantageous, to ensure the safety of the use of the brace in fracture treatment, particularly where increased swelling of the limb may occur.

When the strap 4 is tightened, the compression of the resilient material 7 stores energy in the brace 1. As the fracture swelling subsides, the resilient material 7 reacts by partially (or fully) returning to its original shape, so that any slack caused by the reduction in the swelling is taken up by the expanding resilient material 7. Thus, the resilient material 7 re-expands upon any decrease of swelling in the arm, releasing the stored energy and maintaining pressure on the arm (specifically, on the fracture under the distal load area 5).

Thus, pressure is maintained on the fracture throughout treatment, through the three load areas 5, 6, 13 of the brace 1.

Modifications and improvements may be incorporated without departing from the scope of the invention. For example, the resilient material is not necessarily polyurethane and could alternatively be any suitable resilient material. The resilient material may alternatively comprise a foam.

The brace could have more load areas than the three shown. The antero-ulnar portion 3 could be larger than shown, with a relatively raised load area that is smaller than the whole antero-ulnar portion 3.

The rivets 11 are not essential; the strap 4 is not necessarily permanently attached to the antero-ulnar portion 3 by any means. If the strap 4 is permanently attached, any suitable fixing means could alternatively be used, e.g. stitching or staples. The strap 4 is not necessarily fastened by a buckle 10; any suitable fastening means could alternatively be used.

The brace is not necessarily limited for use only on an arm, but could also be adapted for use on a leg, e.g. for an ankle fracture.

Optionally, the arms of the U-shape of the rigid shell 2 may be longer or shorter than shown.

The resilient material may be attached to the rigid shell 2 by any suitable means, e.g. adhesion, riveting, and/or bonding.

The size and shape of the load areas 5, 6, 13 may be different than illustrated, e.g. the load areas 5, 6, 13 are not necessarily rectangular and could be larger or smaller.

Trials have found that the combination of the use of resilient material 7 on a rigid shell body, and an inelastic strap provided the best fracture loading over a sustained time. However, in some alternative embodiments, the strap 4 may be partially or fully elastic.

## Claims

1. A fracture brace for a limb comprising:
a first portion (2) comprising a rigid shell having at least two load areas (5, 6) for contact with the limb;
a second portion (3) comprising at least one load area (13) for contact with the limb; and
a strap (4) adapted to encircle the first and second portions (2, 3);
wherein the first portion also includes a resilient material (7), **characterised in that** the resilient material (7) is attached to the radially outer part of the shell; and
wherein, in use, the strap (4) overlies and compresses the resilient material (7) so that the fracture brace exerts pressure on the limb, and wherein the resilient material (7) re-expands upon any decrease of swelling in the limb to maintain pressure on the limb.

2. A fracture brace as claimed in claim 1, wherein the strap (4) is inelastic.

3. A fracture brace as claimed in claim 2, wherein the strap (4) has an angle creased in it to allow it to conform to the conical shape of a limb.

4. A fracture brace as claimed in any preceding claim, wherein the first and second portions (2, 3,) are shaped such as to space the entirety of the strap (4) from the limb in use.

5. A fracture brace as claimed in claim 4, wherein the rigid shell is u-shaped in cross-section, the strap (4) extending over and around the u-shape directly to the second portion (3) without touching the limb.

6. A fracture brace as claimed in any preceding claim, wherein the rest of the fracture brace is located radially outwards of the load areas (5, 6, 13) so that, apart from the load areas (5, 6, 13), no other part of the fracture brace contacts the limb in use.

7. A fracture brace as claimed in any preceding claim, wherein each load area (5, 6, 13) comprises an attached preformed pad.

8. A fracture brace as claimed in claim 7, wherein the preformed pads are made of a high friction material adapted to prevent slippage of the brace and thus maintain its position on the limb in use.

9. A fracture brace as claimed in any preceding claim, wherein only two load areas (5,6) are provided on the first portion (2) and only one load area (13) is provided on the second portion (3).

10. A fracture brace as claimed in any preceding claim, wherein the load area (13) on the second portion is located between the two load areas (5, 6) provided on the first portion (2).

11. A fracture brace as claimed in any preceding claim, wherein the strap (4) covers at least a part of the load area (13) on the second portion (3) and extends between the two load areas (5, 6,) provided on the first portion (2).

12. A fracture brace as claimed in any preceding claim, wherein the two load areas (5, 6,) provided on the first portion (2) are spaced away from one another towards opposite ends of the first portion (2).

13. A fracture brace as claimed in any preceding claim, wherein the resilient material (7) is positioned closer to the distal load area (5) than to the proximal load area (6).

14. A fracture brace as claimed in any preceding claim, wherein the resilient material (7) is wider than the strap (4).

15. A fracture brace as claimed in any preceding claim, having a slot on the first portion (2) to accommodate the strap (4) whereby the strap (4) is maintained in a position on the first portion (2) in which the resilient material (7) extends beyond each side of the strap (4) during treatment.

## Patentansprüche

1. Eine Bruchbandage für eine Gliedmaße, beinhaltend:
einen ersten Abschnitt (2), beinhaltend eine steife Hülle mit mindestens zwei Belastungsbereichen (5, 6) für den Kontakt mit der Gliedmaße;
einen zweiten Abschnitt (3), beinhaltend mindestens einen Belastungsbereich (13) für den Kontakt mit der Gliedmaße; und
einen Riemen (4), der angepasst ist, um den ersten und zweiten Abschnitt (2, 3) zu umgeben;
wobei der erste Abschnitt auch ein nachgiebiges Material (7) umfasst,
**dadurch gekennzeichnet, dass** das nachgiebige Material (7) an dem radial äußeren Teil der Hülle angebracht ist; und
wobei der Riemen (4) im Gebrauch auf dem nachgiebigen Material (7) aufliegt und dieses komprimiert, so dass die Bruchbandage Druck auf die Gliedmaße ausübt, und
wobei sich das nachgiebige Material (7) bei jeglicher Abnahme von Schwellung in der Gliedmaße erneut ausdehnt, um den Druck auf die Gliedmaße beizubehalten.

2. Bruchbandage gemäß Anspruch 1, wobei der Riemen (4) unelastisch ist.

3. Bruchbandage gemäß Anspruch 2, wobei in dem Riemen (4) ein Winkel gefaltet ist, um zu ermöglichen, dass er sich an die konische Form einer Gliedmaße angleichen kann.

4. Bruchbandage gemäß einem der vorhergehenden Ansprüche, wobei der erste und zweite Abschnitt (2, 3) derart geformt sind, um im Gebrauch den gesamten Riemen (4) mit Abstand von der Gliedmaße anzuordnen.

5. Bruchbandage gemäß Anspruch 4, wobei die steife Hülle im Querschnitt U-förmig ist, wobei sich der Riemen (4) über und um die U-Form direkt zu dem zweiten Abschnitt (3) hin erstreckt, ohne dabei die Gliedmaße zu berühren.

6. Bruchbandage gemäß einem der vorhergehenden Ansprüche, wobei der Rest der Bruchbandage radial nach außen von den Belastungsbereichen (5, 6, 13) befindlich ist, so dass, abgesehen von den Belastungsbereichen (5, 6, 13), im Gebrauch kein anderer Teil der Bruchbandage mit der Gliedmaße in Kontakt kommt.

7. Bruchbandage gemäß einem der vorhergehenden Ansprüche, wobei jeder Belastungsbereich (5, 6, 13) ein angebrachtes vorgeformtes Pad beinhaltet.

8. Bruchbandage gemäß Anspruch 7, wobei das vorgeformte Pad aus einem Material mit hoher Reibung gefertigt sind, das angepasst ist, um im Gebrauch ein Rutschen der Bandage zu verhindern und somit seine Position auf der Gliedmaße beizubehalten.

9. Bruchbandage gemäß einem der vorhergehenden Ansprüche, wobei nur zwei Belastungsbereiche (5, 6) auf dem ersten Abschnitt (2) bereitgestellt sind und nur ein Belastungsbereich (13) auf dem zweiten Abschnitt (3) bereitgestellt ist.

10. Bruchbandage gemäß einem der vorhergehenden Ansprüche, wobei der Belastungsbereich (13) auf dem zweiten Abschnitt zwischen den zwei auf dem ersten Abschnitt (2) bereitgestellten Belastungsbereichen (5, 6) befindlich ist.

11. Bruchbandage gemäß einem der vorhergehenden Ansprüche, wobei der Riemen (4) mindestens einen Teil des Belastungsbereichs (13) auf dem zweiten Abschnitt (3) abdeckt und sich zwischen den zwei auf dem ersten Abschnitt (2) bereitgestellten Belastungsbereichen (5, 6) erstreckt.

12. Bruchbandage gemäß einem der vorhergehenden Ansprüche, wobei die zwei auf dem ersten Abschnitt (2) bereitgestellten Belastungsbereiche (5, 6) voneinander weg zu entgegengesetzten Enden des ersten Abschnitts (2) mit Abstand angeordnet sind.

13. Bruchbandage gemäß einem der vorhergehenden Ansprüche, wobei das nachgiebige Material (7) näher an dem distalen Belastungsbereich (5) als dem proximalen Belastungsbereich (6) positioniert ist.

14. Bruchbandage gemäß einem der vorhergehenden Ansprüche, wobei das nachgiebige Material (7) breiter als der Riemen (4) ist.

15. Bruchbandage gemäß einem der vorhergehenden Ansprüche, die einen Schlitz auf dem ersten Abschnitt (2) aufweist, um den Riemen (4) aufzunehmen, wodurch der Riemen (4) in einer Position auf dem ersten Abschnitt (2) beibehalten wird, in der sich das nachgiebige Material (7) während der Behandlung über jede Seite des Riemens (4) hinaus erstreckt.

## Revendications

1. Un appareil orthopédique pour fracture destiné à un membre comprenant :
une première portion (2) comprenant une coque rigide ayant au moins deux zones de charge (5, 6) destinée à être en contact avec le membre ;
une deuxième portion (3) comprenant au moins une zone de charge (13) destinée à être en contact avec le membre ; et
une sangle (4) conçue pour encercler les première et deuxième portions (2, 3) ;
dans lequel la première portion inclut également un matériau résilient (7), **caractérisé en ce que** le matériau résilient (7) est attaché à la partie radialement externe de la coque ; et
dans lequel, lors de l'utilisation, la sangle (4) se superpose au matériau résilient (7) et le comprime de sorte que l'appareil orthopédique pour fracture exerce une pression sur le membre, et dans lequel le matériau résilient (7) se re-dilate lors de toute diminution de gonflement dans le membre afin de maintenir une pression sur le membre.

2. Un appareil orthopédique pour fracture tel que revendiqué dans la revendication 1, dans lequel la sangle (4) est inélastique.

3. Un appareil orthopédique pour fracture tel que revendiqué dans la revendication 2, dans lequel la sangle (4) a un angle marqué dans celle-ci pour lui permettre d'épouser la forme conique d'un membre.

4. Un appareil orthopédique pour fracture tel que revendiqué dans n'importe quelle revendication précédente, dans lequel les première et deuxième portions (2, 3) sont formées de telle sorte à espacer l'intégralité de la sangle (4) du membre lors de l'utilisation.

5. Un appareil orthopédique pour fracture tel que revendiqué dans la revendication 4, dans lequel la coque rigide a la forme d'un u en coupe transversale, la sangle (4) s'étendant par-dessus et autour de la forme en u directement jusqu'à la deuxième portion (3) sans toucher le membre.

6. Un appareil orthopédique pour fracture tel que revendiqué dans n'importe quelle revendication précédente, dans lequel le reste de l'appareil orthopédique pour fracture est situé radialement vers l'extérieur des zones de charge (5, 6, 13) de sorte que, en dehors des zones de charge (5, 6, 13), aucune autre partie de l'appareil orthopédique pour fracture n'est au contact du membre lors de l'utilisation.

7. Un appareil orthopédique pour fracture tel que revendiqué dans n'importe quelle revendication précédente, dans lequel chaque zone de charge (5, 6, 13) comprend un coussinet préformé attaché.

8. Un appareil orthopédique pour fracture tel que revendiqué dans la revendication 7, dans lequel les coussinets préformés sont réalisés en un matériau à coefficient de frottement élevé conçu pour empêcher un glissement de l'appareil orthopédique et maintenir ainsi sa position sur le membre lors de l'utilisation.

9. Un appareil orthopédique pour fracture tel que revendiqué dans n'importe quelle revendication précédente, dans lequel seules deux zones de charge (5, 6) sont prévues sur la première portion (2) et seule une zone de charge (13) est prévue sur la deuxième portion (3).

10. Un appareil orthopédique pour fracture tel que revendiqué dans n'importe quelle revendication précédente, dans lequel la zone de charge (13) sur la deuxième portion est située entre les deux zones de charge (5, 6) prévues sur la première portion (2).

11. Un appareil orthopédique pour fracture tel que revendiqué dans n'importe quelle revendication précédente, dans lequel la sangle (4) recouvre au moins une partie de la zone de charge (13) sur la deuxième portion (3) et s'étend entre les deux zones de charge (5, 6) prévues sur la première portion (2).

12. Un appareil orthopédique pour fracture tel que revendiqué dans n'importe quelle revendication précédente, dans lequel les deux zones de charge (5, 6) prévues sur la première portion (2) sont espacées à distance l'une de l'autre vers des extrémités opposées de la première portion (2).

13. Un appareil orthopédique pour fracture tel que revendiqué dans n'importe quelle revendication précédente, dans lequel le matériau résilient (7) est positionné plus près de la zone de charge distale (5) que de la zone de charge proximale (6).

14. Un appareil orthopédique pour fracture tel que revendiqué dans n'importe quelle revendication précédente, dans lequel le matériau résilient (7) est plus large que la sangle (4).

15. Un appareil orthopédique pour fracture tel que revendiqué dans n'importe quelle revendication précédente, ayant une fente sur la première portion (2) pour loger la sangle (4) grâce à quoi la sangle (4) est maintenue dans une position sur la première portion (2) dans laquelle le matériau résilient (7) s'étend au-delà de chaque côté de la sangle (4) durant le traitement.
